# EUROPEAN PATENT APPLICATION

(11) **EP 2 108 957 A1**
(43) Date of publication of application: **14.10.2009**
(21) Application number: 08103413.4
(22) Date of filing: 07.04.2008
(51) Int. Cl.: G01N 33/68, C07K 1/13

(54) **Compounds and methods for the labelling and affinity-selection of proteins**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates to compounds and methods for the isotopic/isobaric labeling and the subsequent affinity selection and analysis of proteinaceous molecules. In particular, the invention relates to a labeling reagent for the labeling of proteinaceous molecules, comprising an isobaric label component, an isotopic label component, and a reactive group capable of reacting with a proteinaceous molecule, wherein the isotopic label component concomitantly is an affinity tag. The invention is also directed to a kit-of-parts comprising a combinatorial plurality of such labeling reagents as well as to a corresponding method for the analysis of proteinaceous molecules, the method comprising labeling at least one subset of the proteinaceous molecules present by employing a kit-of-parts as defined herein and subsequently separating the labeled molecules by affinity purification via the affinity tag comprised in the label. Finally, the invention relates to the uses of such methods for protein expression profiling or proteomic analyses.

## Description

### SUBJECT OF THE INVENTION

The present invention relates to compounds and methods for the separation and subsequent analysis of proteinaceous molecules from complex samples by coupling the isotopic and isobaric double labeling of the proteinaceous molecules to their subsequent affinity purification by means of an affinity tag concomitantly functioning as an isotopic label component.

### BACKGROUND OF THE INVENTION

The identification, separation, and analysis of particular proteins or subsets of proteins from complex samples are invaluable for unraveling how biological processes occur at a molecular level or to which degree proteins differ among various cell types or between physiological states.

A major challenge in modem biology is directed to the understanding of the expression, function, and regulation of the entire set of proteins encoded by an organism, a technical field commonly known as proteomics. However, as there is no possibility to amplify proteins, research in this field is generally rather tedious because even a cell extract of a relatively simple prokaryotic organism contains a multitude of proteins encompassing a huge range of concentrations. Therefore, such a task is beyond the capabilities of any current single analytical methods.

Thus, due to the methodological constraints proteome analysis relies not only to methods for identifying and quantifying proteins but - to a considerable extent - also on methods allowing their accurate and reliable separation according to their structural and/or functional properties, with these subsets being then better accessible to further analysis.

The proteome is of dynamic nature, with alterations in protein synthesis, activation, and/or post-translational modification in response to external stimuli or alterations in the cellular environment. Therefore, the proteome's inherent complexity exceeds that of the genome or the transcriptome the mRNA complement of a cell.

Due to the extraordinary amount of data to be processed in such proteomic studies the protein/peptide identification process demands tremendous resolving power. Two methods commonly used to resolve such highly complex mixtures are two-dimensional gel electrophoresis (2D-GE; cf., e.g., O'Farrell, P.H. (1975) J. Biol. Chem. 250, 4007-4021) and (two-dimensional) liquid chromatography ((2D)-LC; cf., e.g., Lipton, M.S. et al. (2002) Proc. Natl. Acad. Sci. USA 99, 11049-11054). The peptides and proteins isolated by 2D-GE or 2D-LC are usually identified by mass spectrometry or by determining amino acid composition and/or amino acid sequence.

However, although useful for many applications these identification techniques have major drawbacks with regard to proteomic studies, where highly complex samples are to be investigated. For example, hydrophobic membrane proteins, highly basic or acidic proteins, very large or very small proteins are often poorly resolved via 2D-GE. Furthermore, the detection (sensitivity) limits of these methods as well as shortcomings in labeling technology do not allow for the reliable analysis of multiple samples in parallel, e.g., for comparing relative protein levels between different disease groups, different progression stages of a disease, and between disease stages *vs*. healthy controls or for performing high-throughput screening analyses such as protein expression profiling or the identification of protein biomarkers.

Currently, there are several techniques available, for example mass spectrometry, which enable the identification and characterization of particular proteins in a given sample. However, global proteomic studies are generally hampered due to a limited sensitivity of detection. Thus, additional fractionation, enrichment or selection steps (e.g., by affinity purification) which might interfere with further analyses are required in order to reduce the complexity of the sample. Another problem in determining statistically significant assay results is the relative quantification of differences in protein expression between multiple samples. Therefore, it is highly desirable to develop methodologies which overcome the above limitations and enable processing of multiple complex samples in parallel in order to reduce the methodological variability between different individual samples and thus to increase the statistical significance of the assay results obtained.

Recently, two different approaches for protein labeling have been developed in order to address this goal.

The first technique is called Isotope-Coded Affinity Tag (ICAT) technology and allows for quantitative proteomic analysis based on differential isotopic tagging of related protein mixtures (cf. Gygi et al. (1999) Nat. Biotechnol. 17, 994-999). That is, this labeling method employs a set of labels having the same chemical formula but differing from each other in the number and/or type of isotopes present in one or more atoms, resulting in a mass difference. The ICAT reagent described uses three functional elements: a thiol-reactive group for the selective labeling of reduced cysteine residues, a biotin affinity tag to allow for selective isolation of cysteine labeled peptides, and an isotopic tag, which is synthesized in two isotopic forms, the "light" (non-isotopic) or "heavy" (utilizing ²H or ¹³C) forms. As only a comparably moderate number of the proteins in a given sample contains cysteine residues in its primary sequence, resulting in a decreased complexity of the sample which, in turn, facilitates the reliability of subsequent sample processing.

An alternative approach makes use of isobarically labeled reagents referred to as Isobaric Tag for Relative and Absolute Quantitation (iTRAQ; cf. Ross, P.L. et al. (2004) Mol. Cell. Proteomics 3, 1154-1169). This approach employs four different iTRAQ reagents, each containing a reporter group, a balance group and a peptide reactive group which reacts with primary amine groups. The reporter group has a mass of 114, 115, 116 or 117 Da, depending on differential isotopic combinations of ¹²C/¹³C and ¹⁶O/¹⁸O in each reagent. The balance group varies in mass from 31 to 28 Da to ensure that the combined mass of the reporter group and the balance group remains constant (145 Da) for the four reagents. Accordingly, labeling of the same peptide with each of these reagents results in peptides which are isobaric and thus chromatographically indistinguishable from each other (i.e. all contribute to one ion species that is observed in the MS analysis and used for CID). During MS/MS tandem mass spectrometry, however, at least the respective reporter groups are released upon collision-induced dissociation (CID), displaying distinct masses of 114 to117 Da. The intensity of these fragments can be used for quantification of the individual proteins and/or peptides in a single experiment. This results in increased signal intensity and, in turn, an increased probability to correctly identify peptides, particularly low abundance-peptides.

The above described ICAT and iTRAQ techniques allow the multiplexing of either 2 (ICAT) or up to 4 (iTRAQ) individual samples and allows to process them simultaneously thereby minimizing technical variability. Nevertheless, both of these approaches have significant limitations.

The ICAT approach, even though allowing the fast screening of protein expression differences, is restricted to the investigation of only two samples, for example two disease groups, which normally does not represent the full spectrum of disease relevant groups (e.g., different stages of progression of a disease). Therefore, it would be advantageous to have a technique available, which allows the investigation of more than 2 groups.

The iTRAQ technology, on the other hand, while allowing multiple sample analysis, has the clear disadvantage that it requires performing MS/MS scans on each MS signal (for both unlabeled and labeled proteins and/or peptides) for the relative quantification of the signals. In practice, this is not feasible when the starting material is of high complexity such as in human serum, or other body fluids, as the analysis will be very time consuming.

Therefore, it is a continuous need for improved labeling reagents for proteinaceous molecules and for corresponding labeling methods that overcome the above limitations and enable the parallel processing of multiple complex samples. In particular, it would be desirable to provide labeling methods which concomitantly allow for the subsequent selective purification and analysis of the labeled proteinaceous molecules.

### OBJECTIVE AND SUMMARY OF THE INVENTION

It is an objective of the present invention to provide novel compounds and corresponding methods for the labeling and the subsequent separation and analysis of proteinaceous molecules from complex samples. More specifically, it is an object of the present invention to provide methods for performing multiple such analyses in parallel.

This objective as well as others which will become apparent from the ensuing description are attained by the subject matter of the independent claims.

Some of the preferred embodiments of the present invention are defined by the subject matter of the dependent claims.

In a first aspect, the present invention relates to a labeling reagent for the labeling of proteinaceous molecules, comprising:
(a) an isobaric label component;
(b) an isotopic label component; and
(c) a reactive group capable of reacting with a proteinaceous molecule;
wherein the isotopic label component concomitantly is an affinity tag.

In one embodiment, the labeling reagent has a configuration, in which the isotopic label component/affinity tag is arranged between the isobaric label component and the reactive group.

In another embodiment, the labeling reagent has a configuration, in which the isobaric label component is arranged between the isotopic label component/affinity tag and the reactive group.

In a preferred embodiment, the affinity tag is selected from the group consisting of (His)₆ tag, (His)₄ tag, (His)₃ tag, (His)₂ tag, (Leu)₄ tag, (Leu)₃ tag, (Leu)₂ tag, c-Myc tag, HA tag, FLAG tag, VSV-G tag, HSV tag, V5 tag, biotin and derivatives thereof, carbohydrates, and glycans.

In a further preferred embodiment, the reactive group is selected from the group consisting of amino-reactive groups, sulfhydryl-reactive groups, and carboxyl-reactive groups.

In a second aspect, the present invention relates to a kit-of-parts, comprising a combinatorial plurality of n·m labeling reagents as defined herein, wherein the integer n is ≥ 2 and represents the number of differentially labeled isobaric label components, and wherein the integer m is ≥ 2 and represents the number of differentially labeled isotopic label components/affinity tags.

Preferably, the kit-of-parts comprises n·m labeling reagents, wherein the integer n is ≥ 4 and the integer m is ≥ 2.

In a third aspect, the present invention relates to a method for the analysis of one or more proteinaceous molecules in one or more samples, comprising:
(a) providing a kit-of-parts as defined herein, the kit-of-parts comprising a combinatorial plurality of n·m labeling reagents;
(b) labeling at least one subset of the proteinaceous molecules comprised in the one or more samples by individually contacting each of the one or more samples with another one of the n·m labeling reagents;
(c) combining the one or more samples;
(d) separating the labeled at least one subset proteinaceous molecules by affinity purification via the affinity tag comprised in the label; and
(e) analyzing the separated at least one subset of proteinaceous molecules.

In a preferred embodiment, the method is performed with M·N samples, wherein the integer M represents the number of groups of samples and the integer N represents the number of individual members of each group of samples, and wherein N = n and M = m.

In one embodiment, the method further comprises cleaving the proteinaceous molecules into peptides prior to performing step (c).

In another embodiment, the method further comprises fractionating the separated at least one subset of proteinaceous molecules prior to performing step (f).

In a preferred embodiment of the inventive method, the analysis of the separated at least one subset of proteinaceous molecules is performed by means of mass spectrometry.

In another preferred embodiment, the method further comprises: comparing the results of the analyses obtained in step (f) for each of the one or more samples. Particularly preferably, the method is performed in a high-throughput format.

In a final aspect, the present invention relates to the uses of the method as defined herein for performing protein expression profiling or for performing proteomic analyses.

Other embodiments of the present invention will become apparent from the detailed description hereinafter.

### FIGURE LEGENDS

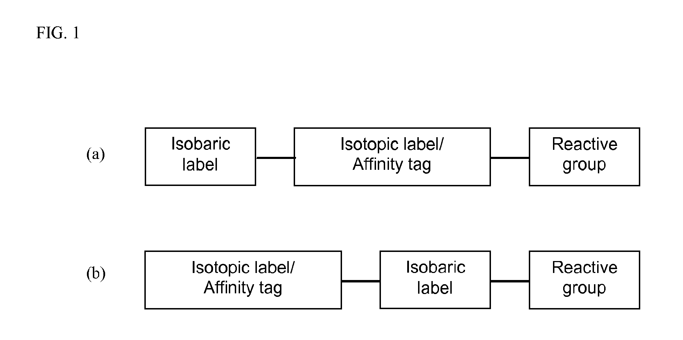

- Fig. 1: depicts a schematic illustration of two representative embodiments of the labeling reagent of the invention. In configuration (a) the isotopic label component/affinity tag is arranged between the isobaric label component and the reactive group. In configuration (b) the isobaric label component/affinity tag is arranged between the isotopic label component/affinity tag and the reactive group.

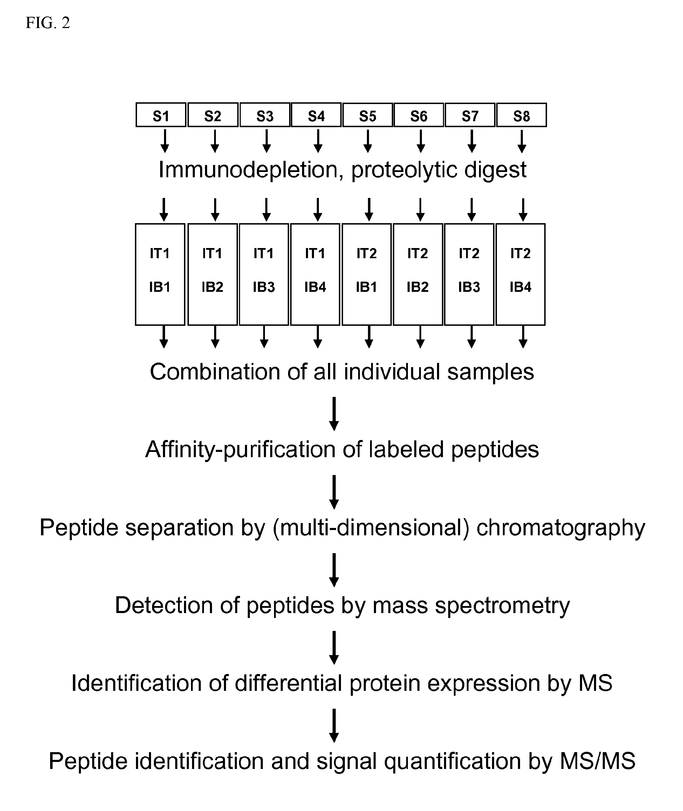

- Fig. 2: depicts a schematic illustration of the application of the method of the present invention for the analysis of two different groups of samples (e.g. representing a disease stage and a healthy control), each group comprising four members (e.g. representing different patients and healthy subjects, respectively). The eight individual samples are designated S1 to S8 (S1 to S4 represent the first group of samples, and S5 to S8 represent the second group of samples). After sample processing (e.g., depletion of highly abundant proteins and/or proteolytic cleavage) each sample is labeled with a different labeling reagents of the invention. For the first group of samples the isotopic label 1 ("IT1") is used, whereas for the second group of samples the isotopic label 2 is used ("IT2"). Importantly, IT1 and IT2 concomitantly are affinity tags. The two groups of samples can thus be discriminated by a characteristic mass difference in a mass spectrometric (MS) analysis. The individual members within the groups of samples are labeled with different isobaric labels (designated "IB1" to "IB4"), respectively. Therefore, the four members of each group of samples can be discriminated in a tandem MS/MS analysis. Subsequently, the individual samples are combined and the labeled proteins and/or peptides are purified via the affinity tag comprised in the label (i.e. IT1 and IT2) by affinity chromatography. The purified proteins and/or peptides are separated, optionally fractionated and analyzed by mass spectroscopy. By comparing the results of the two groups of samples differences in protein expression can be determined.

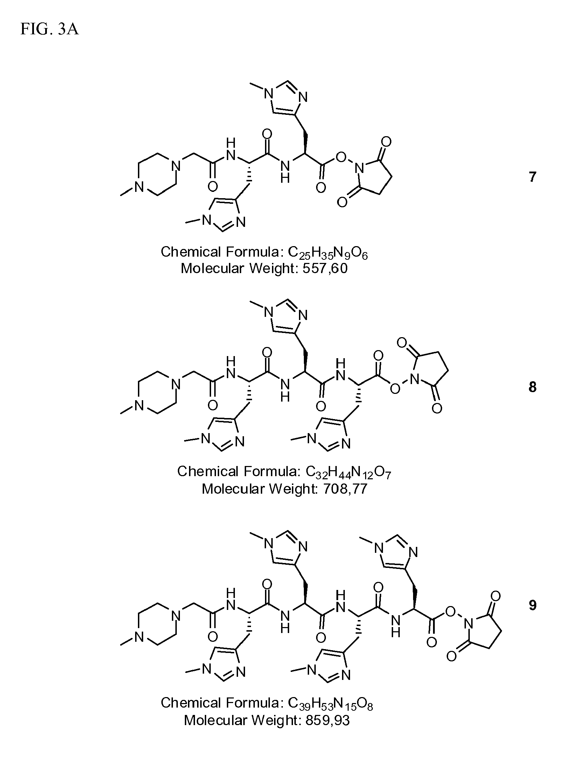

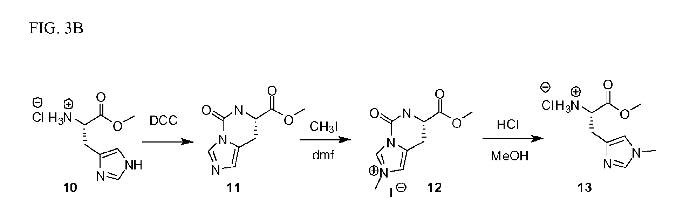

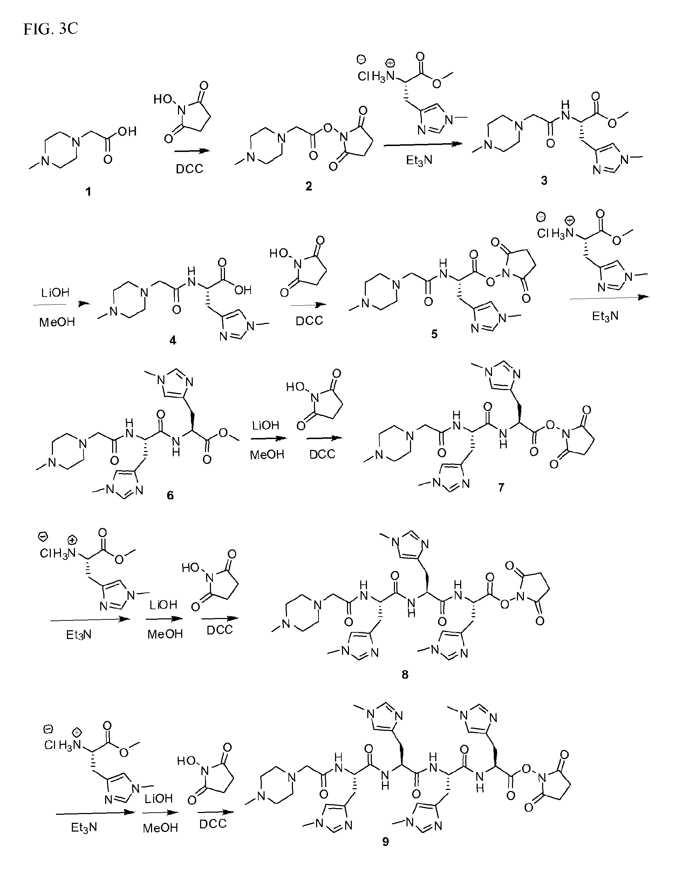

- Fig. 3: depicts representative oligo-histidine (His) compounds to be employed as isotopic labels/affinity tags in the labeling reagents of the invention. **Fig 3A** shows the chemical structures of (His)₂ (7), (His)₃ (8), and (His)₄ (9) compounds which may be synthesized using different isotopes in one or more atoms. **Fig 3B** shows the methylation of the histidine side chain as the initial step of the synthesis pathway ( cf. Recueil Trav. Chim. Pays-Bas (1978) 97, 281). Finally, **Fig. 3C** schematically illustrates the synthesis pathway for the three compounds shown in Fig. 3A.

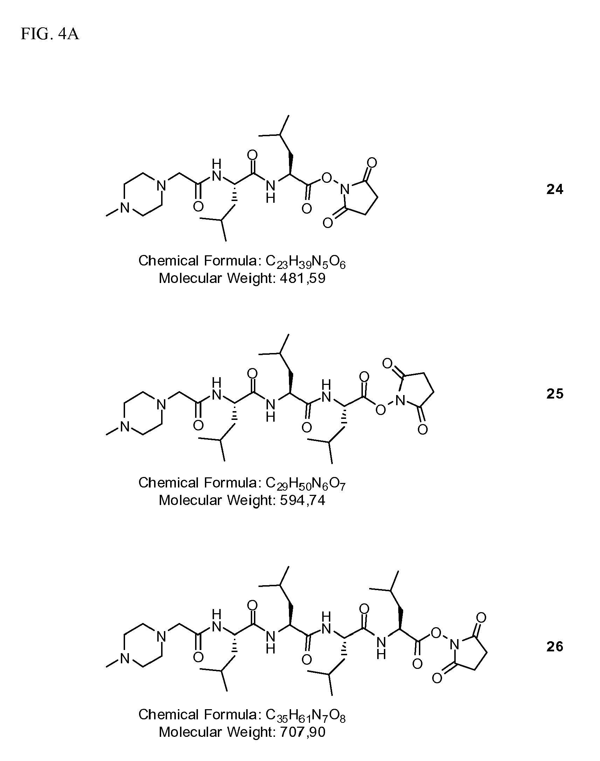

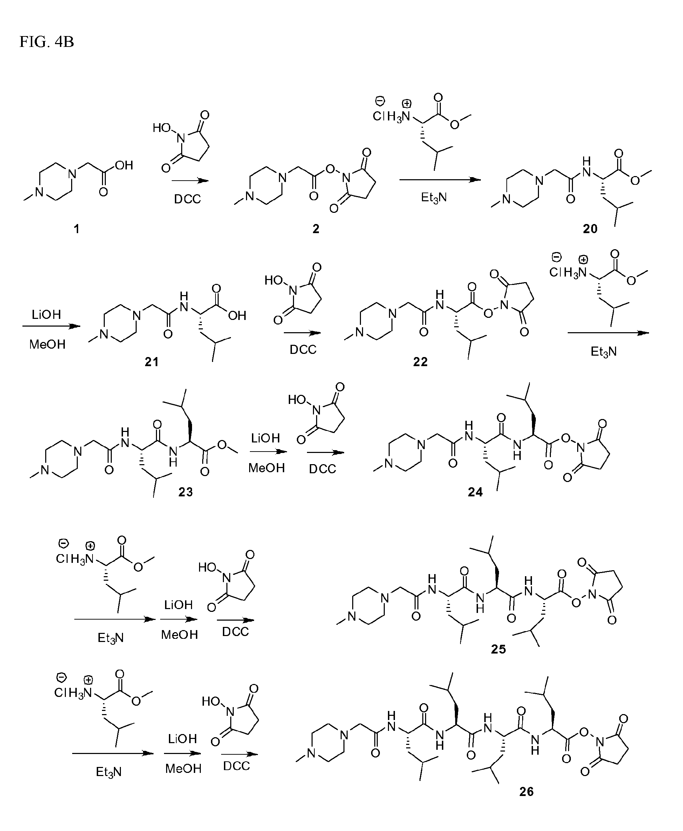

- Fig. 4: depicts representative oligo-leucine (Leu) compounds to be employed as isotopic labels/affinity tags in the labeling reagents of the invention. **Fig 4A** shows the chemical structures of (Leu)₂ (24), (Leu)₃ (25), and (Leu)₄ (26) compounds which may be synthesized using different isotopes in one or more atoms. **Fig 4B** schematically illustrates the synthesis pathway for the three compounds shown in Fig. 4A.

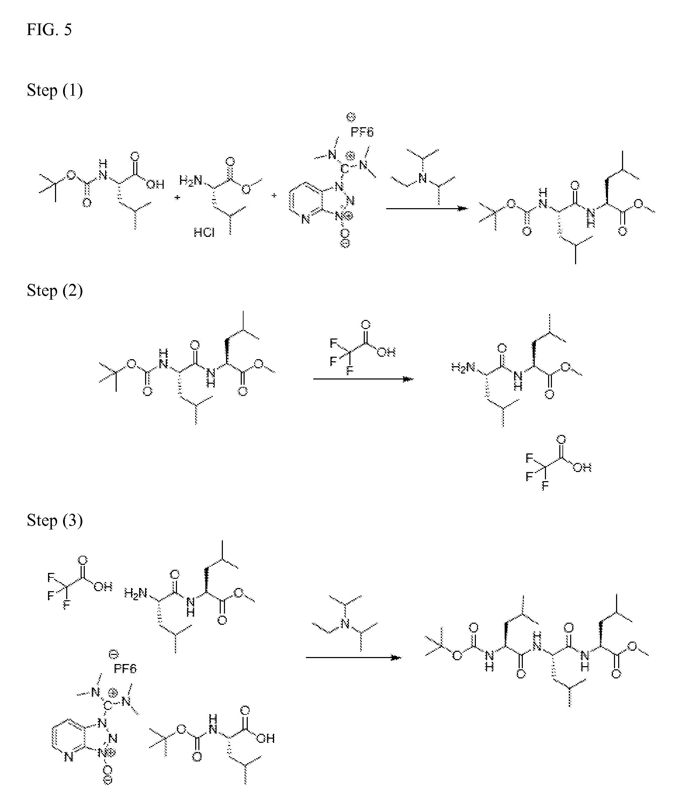

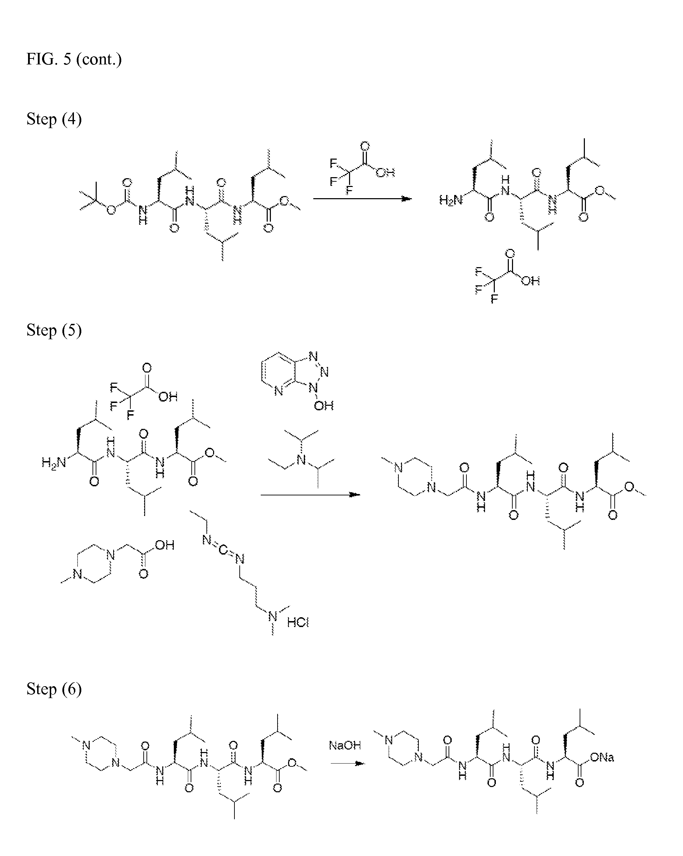

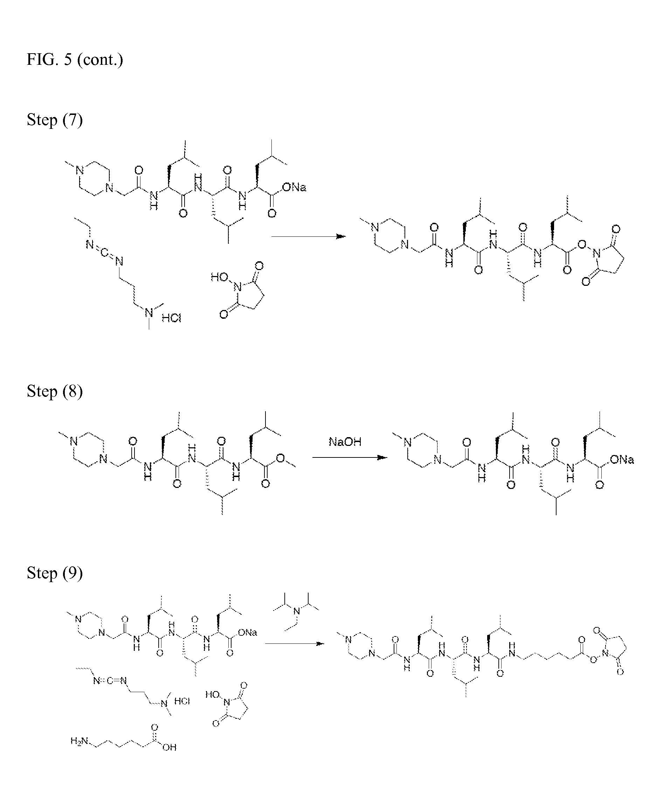

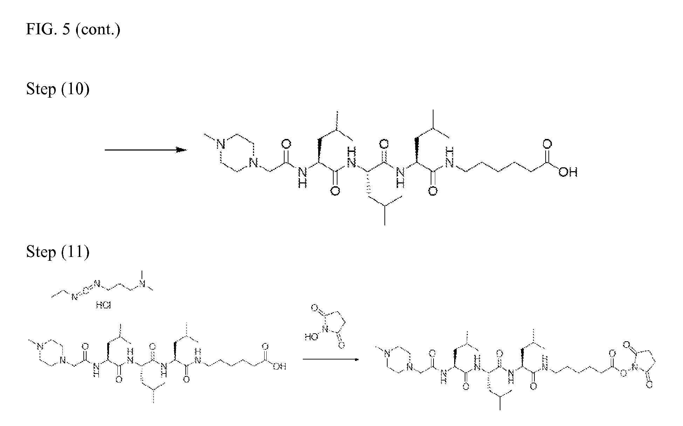

- Fig. 5: depicts the detailed reaction steps for the chemical synthesis of the (Leu)₃ compound shown in Fig. 4A. The synthesis protocol is outlined in Example 1.

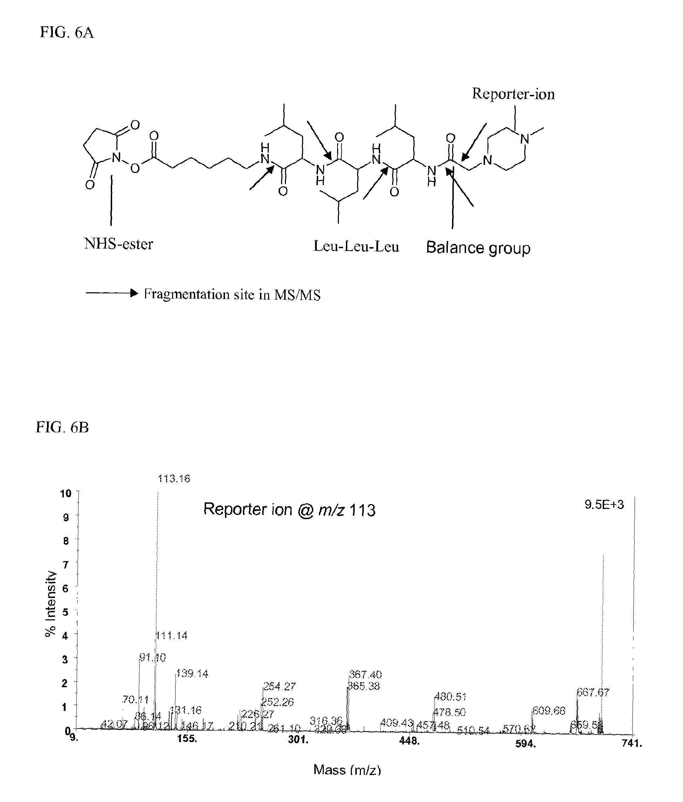

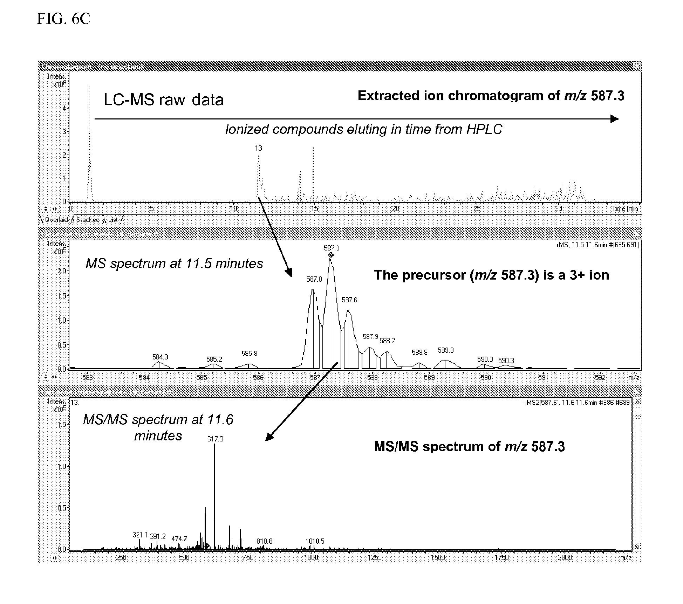

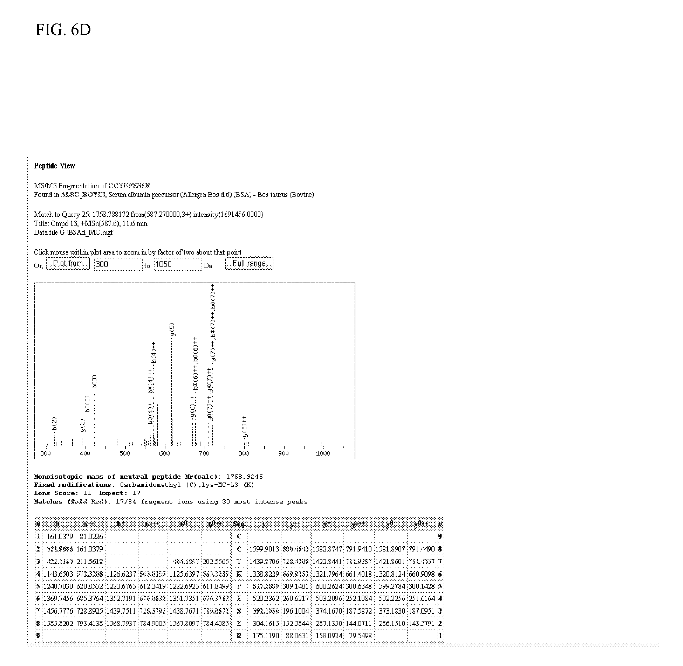

- Fig. 6: depicts a pilot experiment using the (Leu)₃ compound, as synthesized in Fig. 5 for labeling bovine serum albumin (BSA). **Fig. 6A** schematically illustrates the (Leu)₃ compound comprising both the reporter ion and the balance group of the isobaric label component as well as a NHS-ester as the reactive group. Labeling and trypsin-digestion of BSA was performed according to Example 2. As a quality control, labeled fragment release was analyzed by MALDI MS/MS analysis (**Fig. 6B**). For peptide identification, the digested BSA peptides were fractionated (sorted) using reversed phase liquid chromatography-electrospray ion trap mass spectrometry (LC-MS; cf. **Fig. 6C**, top panel). A representative LC-MS peak was further analyzed using MS (**Fig. 6C****,** middle panel).A representative MS peak corresponding to a labeled peptide was subsequently subjected to MS/MS analysis (**Fig. 6C**, bottom panel). The resulting MS/MS spectra were then converted to an mgf-file (Mascot Generic Format) which was searched against the SwissProt database using the Mascot software. Finally, the amino acid sequence (single-letter code) of the peptide analyzed was determined to be CCTKPESER (**Fig. 6D**).

### DETAILED DESCRIPTION OF THE INVENTION

The present invention is based on the unexpected finding that the use of labeling reagents combining an isobaric label component and an isotopic label component, the latter one concomitantly functioning as an affinity tag, allows for a simple one-step labeling protocol for protein and/or peptides, followed by the selective affinity purification of the labeled proteinaceous molecules from complex samples.

The specific design of the labeling reagents having a common isotopic label/affinity tag component results in the generation of molecules having a simplified chemical structure and a considerably smaller molecular weight compared to their counterparts comprising the affinity tag as an independent additional entity. Furthermore, this design also allows for omitting a cleavable linker in order to cleave off the affinity tag after protein/peptide purification and finally also results in less complex cost-efficient synthesis of the labeling reagents.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

In one embodiment, the present invention relates to a labeling reagent for the labeling of proteinaceous molecules, comprising:
(a) an isobaric label component;
(b) an isotopic label component; and
(c) a reactive group capable of reacting with a proteinaceous molecule;
wherein the isotopic label component concomitantly is an affinity tag.

The term "proteinaceous molecules", as used herein, refers to any naturally occurring or synthetic (e.g., generated by chemical synthesis or recombinant DNA technology) macromolecules comprising a plurality of natural or modified amino acids connected via a peptide bond. The length of such a proteinaceous molecule may vary from two to several thousand amino acids (the term thus also includes what is generally referred to as oligopeptides). Typically, the term "proteinaceous molecules" relates to proteins having a length of more than 20 amino acids.

The "proteins" to be analyzed in the present invention may have a length from about 30 to about 2500 amino acids, from about 50 to about 1000 amino acids or from about 100 to about 1000 amino acids.

The term "peptide", as used herein, refers to any fragments of the above "proteinaceous molecules" that are obtained after cleavage of one or more peptide bonds. A peptide as used in the present invention is not limited in any way with regard to its size or nature. Typically, peptides to be analyzed in the present invention may have a length from about 2 to about 20 amino acids, from about 3 to about 18 amino acids or from about 5 to about 15 amino acids.

The term " labeling", as used herein, denotes the (chemical) attachment or incorporation of a "label" (i.e. a detectable marker) into a proteinaceous molecule used in the invention.

The term "label", as used herein, refers to any moiety that comprises one or more appropriate chemical substances or enzymes (which are herein denoted "label components"), which directly or indirectly generate a detectable compound or signal in a chemical, physical or enzymatic reaction. If at least two label components are present in a given label, based on its particular properties each label component may be differentially detectable. Within the scope of the present invention, the term "label" is to be understood to refer to the moiety that is bound to the proteinaceous molecule. The moiety prior to the binding to the proteinaceous molecule is denoted herein "labeling reagent".

A label used in the present invention may be attached to an amino acid residue of a protein and/or peptide via a covalent or a non-covalent linkage. Typically, the linkage is a covalent linkage. According to the present invention, this linkage is achieved via a "reactive group", that is a chemical function comprised in the labeling reagent that is capable of reacting with a proteinaceous molecule. In preferred embodiments, the reactive group is selected from the group consisting of amino-reactive groups (i.e. a chemical group reacting with an NH₂ amino-group), sulfhydryl-reactive groups (i.e. a chemical group reacting with an SH sulfhydryl-group), and carboxyl-reactive groups (i.e. a chemical group reacting with an COOH carboxyl-group).

The labels/labeling reagents of the invention typically comprise two label components (i.e. are "double labeled"), an isobaric label component and an isotopic label component, respectively. However, it is also within the scope of the invention that the labels/labeling reagents comprise one or more additional label components. These one or more additional label components may be isobaric label components and/or isotopic label components as well or may be any other label component known in the art including *inter alia* enzyme labels, colored labels, fluorescent labels, chromogenic labels, luminescent labels, radioactive labels, haptens, biotin, metal complexes, metals, and colloidal gold.

In case, one or more such additional label components are present in a labeling reagent according to the invention, said labeling reagent may further comprise a cleavable moiety for specifically cleaving off/removing the one or more additional label components prior to subjecting the labeled proteinaceous molecules to further analysis. The cleavable moiety may be selected from the group consisting of an acid labile moiety, a base labile moiety, a moiety which is cleavable by UV irradiation, a moiety which is cleavable by microwave irradiation, a moiety which is cleavable by a change of electric potential, a moiety which is cleavable by oxidation, a moiety which is cleavable by reduction, a moiety which can be altered by olefine metathesis, and a moiety which can be altered by disulfide exchange (i.e. disulfide moieties). In preferred embodiments, the cleavable moiety is selected from disulfide moieties and acid labile moieties.

The present invention is based on the use of labeling reagents combining isotopic label components and isobaric label components in order to enable multiplexed protein analysis, that is for performing multiple analyses in parallel, for example 4, 8, 16 or more parallel samples, or even high-throughput assays. As outlined above, the mere use of isotopic labels would only allow the parallel processing of two samples. In particular, such a combined labeling strategy employing isotopic and isobaric label components also affords the comparison of relative protein levels between different samples. Accordingly, only those proteins and/or peptides need to be specifically analyzed, for example by tandem MS/MS analysis, for which a differential expression level is observed, thus resulting in a faster and less complex sample analysis.

The term "isobaric label components" (also referred to as "isobaric tags"), as used herein, denotes a set of detectable moieties having the same structure and the same mass, which upon fragmentation release particular fragments having - due to a differential distribution of isotopes within the isobaric labels - the same structure but differ in mass. Isobaric labels typically comprise a reporter group and a balance group, which are separated upon fragmentation and which display a differential distribution of isotopes. In mass spectrometric analyses, the reporter group generates a strong signature ion upon collision induced dissociation (CID, i.e. fragment release). The signature ions individually differ in mass within a set of isobaric label components, as a result of a differential occurrence of one or more isotopes (e.g., ¹²C/¹³C, ¹⁴N/¹⁵N, ¹⁶O/¹⁸O). The balance group comprises a certain compensating number of isotopes so as to ensure that the combined mass of the reporter group and balance group is constant for the different isobaric labels. The balance group may or may not be released from the label upon CID. The molecular weight of an isobaric label component of the invention is between 20 Da and 2500 Da, preferably between 50 Da and 2000 Da, and most preferably between 100 Da and 1500 Da.

Preferred isobaric label components according to the invention include *inter alia* Isobaric Tag for Relative and Absolute Quantitation (iTRAQ) label componentss (cf. Ross, P.L. et al. (2004) Mol. Cell. Proteomics 3, 1154-1169). This labeling approach employs four different iTRAQ reagents, each containing a reporter group, a balance group and a peptide reactive group which reacts with primary amine groups (for example, the ε amino-group of lysine amino acid residues). The reporter group has a mass of 114, 115, 116 or 117 Da, depending on differential isotopic combinations of ¹²C/¹³C and ¹⁶O/¹⁸O in each reagent. The balance group varies in mass from 28 to 31 Da to ensure that the combined mass of the reporter group and the balance group remains constant (145 Da) for the four reagents. Accordingly, labeling of the same peptide with each of these reagents results in peptides which are isobaric and thus co-elute, for example, in liquid chromatography and consequently are chromatographically indistinguishable from each other. During mass spectrometry, however, at least the respective reporter groups are released upon CID, displaying distinct masses of 114 to117 Da. The intensity of these fragments can be used for quantification of the individual proteins and/or peptides in a single.

The term "isotopic label components" (also referred to as "isobaric tags"), as used herein, denotes a set of detectable moieties having the same chemical formula but differing from each other in the number and/or type of isotopes present of one or more atoms, resulting in a difference in mass of the proteinaceous molecules labeled that can be detected, for example, via mass spectrometry. In other words, otherwise identical proteins and/or peptides labeled with different isotopic labels (e.g., ¹²C/¹³C, ¹⁴N/¹⁵N, ¹⁶O/¹⁸O) can be differentiated as such based on difference in mass. While isobaric labels (see above) in principle constitute a specific type of isotopic labels, in the context of the present invention, the term isotopic label will be used to refer to labels which are not isobaric, but can as such be differentiated based on their molecular weight.

Within the present invention, the isotopic label components concomitantly are affinity tags. The term "affinity tag", as used herein, denotes any chemical moiety that due to its (reversible) binding affinity for a particular matrix (e.g., a solid support, a chromatography resin, a matrix or an antibody) allow the separation of the molecules to which it has been attached by means of an affinity purification method (see below). Thus, in other words, an isotopic label component of the present invention is an affinity tag comprising at one or more atoms different isotopes. Thus, the isotopic label components used In the present invention differ, e.g., from the known Isotope-Coded Affinity Tag (ICAT) labels (cf. Gygi, S.P. et al. (1999) Nat. Biotechnol. 17, 994-999) in that they do not comprise the affinity tag as an independent additional entity.

Typically, an isotopic label component of the invention is synthesized in two isotopic forms, the "light" form (utilizing, e.g. ¹²C, ¹⁴N or ¹⁶O) and the "heavy" form (utilizing, e.g., ¹³C, ¹⁵N or ¹⁸O). The molecular weight of an isotopic label component of the invention is between 100 Da and 3000 Da, preferably between 100 Da and 1500 Da.

Any affinity tag may be employed for the synthesis of an isotopic label component of the invention. Examples of such affinity tag include *inter alia* small chemical compounds (such as biotin and derivatives thereof) and short amino acid sequences, typically 2 to 20 amino acids in length, and preferably 4 to 12 amino acids in length (such as the (His)₆ tag, (Leu)₃ tag, the FLAG tag or the c-Myc tag). All these affinity tags are well established in the art and commercially available.

In preferred embodiments, the affinity tag is selected from the group consisting of (His)₆ tag, (His)₄ tag, (His)₃ tag, (His)₂ tag, (Leu)₄ tag, (Leu)₃ tag, (Leu)₂ tag, c-Myc tag, HA tag, FLAG tag, VSV-G tag, HSV tag, V5 tag, biotin and derivatives thereof, carbohydrates, and glycans. Particularly preferred affinity tags of the invention are selected from the group consisting of (His)₆ tag, (His)₄ tag, (His)₃ tag, (His)₂ tag, (Leu)₄ tag, (Leu)₃ tag, and (Leu)₂ tag.

Since for generating several isotopic variants of the same molecular structure having an equivalent difference in mass, it may be necessary to use different building blocks in the synthesis pathway having potentially different functional groups and reaction mechanisms, different purity or a variable atomic enrichment of the isotope exchanges. This requires many different reaction steps making the synthesis of such a label component laborious, time-consuming and of the also rather expensive.

Thus, in specific embodiments of the present invention isotopic label components are employed which have been synthesized starting from a common building block used as a precursor by repeating the same reaction scheme several times depending on the size of the desired length of the label component. Examples of such building blocks include *inter alia* amino acids and sugar molecules that are commercially available in high purity as well as in different isotopic variants with high atomic enrichment. When employing such building blocks as precursors the synthesis of a set of isotopic label components according to the present invention results in a much simplified, and thus faster and cost-saving reaction scheme. A preferred isotopic label component of the invention that can be prepared in such manner is the (His)₆ tag.

In analogy, the isobaric label components of the present invention may also be synthesized by employing common building blocks. The reporter group may, for example, be based on N-methylpiperazine, whereas the balancing group may be based on, e.g., amino acids or sugar molecules.

The different components of a labeling reagent of the present invention may be arranged in any configuration that allows the reactive group to bind to a proteinaceous molecules. The individual components (i.e. isobaric label component, isotopic label component/affinity tag, and reactive groups) may be directly connected to each other or separated by linker sequences. They may be arranged in a linear manner, as exemplified by the two embodiments schematically illustrated in Fig. 1, or in a branched form.

In preferred embodiments, the labeling reagent has a (linear) configuration, in which the isotopic label component/affinity tag is arranged between the isobaric label component and the reactive group (cf. Fig. 1 (a)). In other preferred embodiments, the labeling reagent has a (linear) configuration, in which the isobaric label component is arranged between the isotopic label component/affinity tag and the reactive group (cf. Fig 1 (b)).

An example of a branched configuration of a labeling reagent of the invention is an arrangement, wherein the isobaric label component is connected to the reactive group via a linker sequence to which the isotopic label component/affinity tag is attached. Another example of such a branched configuration is the reverse arrangement, wherein the isobaric label component is attached to a linker sequence which connects the isotopic label component/affinity tag and the reactive group. A third example of such a branched configuration is an arrangement, wherein the isobaric label component is connected to the isotopic label component/affinity tag via a linker sequence to which the reactive group is attached.

In another embodiment, the present invention relates to a kit-of-parts comprising a plurality of different labeling reagents as defined herein. Particularly, the present invention relates to a kit-of-parts, comprising a combinatorial plurality of n·m labeling reagents as defined herein, wherein the integer n is ≥ 2 and represents the number of differentially labeled isobaric label components/affinity tags, and wherein the integer m is ≥ 2 and represents the number of differentially labeled isotopic label components.

The term "combinatorial plurality", as used herein, denotes that the plurality of labeling reagents comprises the highest number of permutations that can be achieved by combining n different isotopic label components/affinity tags and m different isobaric label components.

For example, when combining four different isobaric label components, IB1, IB2, IB3, and IB4 (i.e. n = 4) with two different isotopic label components/affinity tags IT1 and IT2 (i.e. m = 2) a combinatorial plurality, as defined herein, comprises 4·2 = 8 different combinations: IT1/IB1, IT1/IB2, IT1/IB3, IT1/IB4, IT2/IB1, IT2/IB2, IT2/IB3, and IT2/IB4.

In preferred embodiments, the combinatorial plurality of n·m labeling reagents comprises at least four different isobaric label components (i.e. n is ≥ 4) and at least two different isotopic label components/affinity tags (i.e. m is ≥ 2). Accordingly, kits-of-parts of the present invention may, for example, comprise a combinatorial plurality achieved by combining 4, 8, 12 or 16 different isobaric label components with 2, 3, 4, or 6 different isotopic label components/affinity tags.

In a further embodiment, the present invention relates to a method for the analysis of one or more proteinaceous molecules in one or more samples, comprising:
(a) providing a kit-of-parts as defined in any of claims 5 to 7, the kit-of-parts comprising a combinatorial plurality of n·m labeling reagents;
(b) labeling at least one subset of the proteinaceous molecules comprised in the one or more samples by individually contacting each of the one or more samples with another one of the n·m labeling reagents;
(c) combining the one or more samples;
(d) separating the labeled at least one subset proteinaceous molecules by affinity purification via the affinity tag comprised in the label; and
(e) analyzing the separated at least one subset of proteinaceous molecules.

A schematic illustration of the application of the method according to the invention for the analysis of two different groups of samples (e.g. representing a disease stage and a healthy control), each group comprising four members (e.g. representing different patients and healthy subjects, respectively) is shown in Fig. 2.

The term "sample", as used herein, is not intended to necessarily include or exclude any processing steps prior to the performing of the methods of the invention. The samples can be unprocessed ("crude") samples, extracted protein/peptide fractions, purified protein/peptide fractions and the like. For example, the samples employed may be preprocessed by immunodepletion of one or more subsets of (highly) abundant proteins. Suitable samples include samples of prokaryotic (e.g., bacterial, viral samples) or eukaryotic origin (e.g., fungal, yeast, plant, invertebrate, mammalian and particularly human samples).

The term "complex sample", as used herein, denotes the fact that a sample analyzed using a method of the present invention typically includes a multitude of different proteins and/or peptides (or different variants of such proteins and/or peptides) present in different concentrations. For example, complex samples within the present invention may include at least about 500, at least about 1000, at least about 5000 or at least about 10000 proteins and/or peptides. Typical complex samples used in the invention include *inter alia* cell extracts or lysates of prokaryotic or eukaryotic origin as well as human or non-human body fluids such as whole blood, serum, plasma samples or the like.

The method may be performed with a single sample but typically two or more samples are processed in parallel. In some embodiments, the method is performed with M·N samples, wherein the integer M represents the number of groups of samples (e.g., a disease stages and a healthy control or different progression stages of a disease) and the integer N represents the number of individual members of each group of samples (e.g., samples from different patients or healthy subjects), and wherein N = n and M = m (see above).

In preferred embodiments, the method further comprises cleaving the proteinaceous molecules into peptides prior to subjecting them to the labeling protocol (i.e. prior to performing step (b) of the method according to the invention). Such cleaving of proteins may either be achieved chemically (e.g., via acid or base treatment employing chemicals such as cyanogen bromide, 2-(2'-nitrophenylsulfonyl)-3-methyl-3-bromo-indolenine (BNPS), formic acid, hydroxylamine, iodobenzoic acid, and 2-nitro-5-thiocyanobenzoid acid) or enzymatically via proteases (including *inter alia* trypsin, pepsin, thrombin, papain, and proteinase K), both procedures well known in the art.

The individual samples to be analyzed are labeled by separately contacting each sample with a different labeling reagent of the invention comprised in a kit-of-parts comprising a combinatorial plurality of n·m labeling reagents to ensure a specific labeling of each sample. The labels are attached to at least a subset of the proteinaceous molecules comprised in the one or more samples via the reactive group comprised in the labeling reagents. Subsequently, the one or more samples are combined for further analysis.

The term "at least one subset of the proteinaceous molecules", as used herein, is to be understood in such a way that it may relate - depending on sample processing, particular labeling protocol or the like - to the totality of proteinaceous molecules present in a given sample or a particular part thereof.

Afterwards, the labeled at least one subset proteinaceous molecules comprised in the combined samples is separated by affinity purification via the affinity tag comprised in the label attached to the proteinaceous molecules. The term "affinity purification", as used herein, denotes any purification method (e.g., affinity column chromatography or corresponding batch setups) for the separation of proteinaceous molecules in a sample (mobile phase) that is based on differences in binding interaction with a matrix that is immobilized to a stationary material (solid phase). Selective binding of the proteinaceous molecules to the matrix is typically accomplished via a label (i.e. the affinity tag) displaying specific binding activity for the matrix. Typically, the material to be used as an affinity matrix is insoluble in the system in which the target molecule is found. Examples of suitable matrices include *inter alia* avidin or streptavidin (in case, biotin is used as an affinity tag), Ni²⁺-chelated NTA (in case, (His)₆ is used as an affinity tag) or anti-tag antibodies (i.e. immunoaffinity chromatography) that can be coupled to a support (e.g., anti-FLAG antibodies, anti-(His) antibodies, anti-(Leu) antibodies). All these affinity matrices (i.e. resins and/or anti-tag antibodies) are well established in the art and commercially available.

Finally, the separated labeled proteinaceous molecules are subjected to further qualitative and/or quantitative analysis (i.e. protein/peptide identification and determining their absolute and/or relative concentration in the one or more samples analyzed).

In some embodiments, the separated (label) at least one subset of proteinaceous molecules are further fractionated prior to further analysis (i.e. prior to performing step (e) of the method according to the invention).

The term "fractionation", as used herein, refers to any type processing step in which the proteins and/or peptides present in a sample in a certain quantity are divided (i.e. sorted) up in a large number of smaller quantities (i.e. fractions) according to any differences in their physico-chemical properties such as the molecular mass, their size, and their overall net charge. A common trait in fractionations is the need to find an optimum between the amount of fractions collected and the desired purity in each fraction. Fractionation makes it possible to isolate more than two components in a mixture in a single run. This property sets it apart from other separation techniques.

There are several methods for fractionating proteins and/or peptides well established in the art including classical SDS polyacrylamide gel electrophoresis (SDS-PAGE), two-dimensional gel electrophoresis, size-exclusion chromatography, (two-dimensional) liquid chromatography, and isoelectric focusing, with the latter one being particularly preferred. Methods for analysis, particularly visualization, of the proteinaceous molecules after fractionation has taken place are well established in the art. Other methods of fractionation and combinations thereof may also be envisaged. Thus, fractionation/re-fractionation may rely on ion exchange chromatography, size exclusion chromatography, hydrophobic interaction chromatography, reversed-phase chromatography and/or affinity chromatography.

In other embodiments, when the analysis is performed with more than one sample, the method further comprises: comparing the results of the analyses obtained in for each of the one or more samples in order to determine relative (protein expression) differences between individual samples.

In preferred embodiments, the analysis of the separated at least one subset of proteinaceous molecules is performed by means of mass spectrometry, an analytical technique used to measure the mass-to-charge ratio of ions. The particular mass spectrometric analysis applied may depend on the levels of protein and/or peptide expression determined in different samples. In some embodiments, the method of the invention is performed in a high-throughput format.

In a further embodiment, the invention relates to the use of a labeling agent, a kit-of-parts and/or a method, as described herein, for performing protein expression profiling or for performing proteomic analyses.

While the above invention has been described with respect to some of its preferred embodiments, this is in no way to limit the scope of the invention. The person skilled in the art is clearly aware of further embodiments and alterations to the previously described embodiments that are still within the scope of the present invention.

### EXAMPLES

### Example 1 - Synthesis of a (Leu)₃ labeling compound

The (Leu)₃ labeling compound shown in Figs. 4A and 6A was chemically synthesized according to the following protocol. The individual synthesis steps correspond to the reaction schemes depicted in Fig. 5.
Step (1): The starting materials (S)-2-(*tert*-butoxycarbonylamino)-4-methylpentanoic acid, (S)-methyl-2-amino-4-methylpentanoate hydrochloride, 1-(bis(dimethylamino)methylene)-1H-[1,2,3]triazolo[4,5-b]pyridine-1-ium-3-oxide hexafluorophosphate (V), and N-ethyl-N-isopropylpropan-2-amine were dissolved in dichloromethane (150 ml), 2.0 ml DMF were added and the mixture was stirred for 2 h, after which the Boc protected amine had disappeared (TLC eluens EtOAc/heptane 1:1). 150 ml of saturated ammoniumchloride were added, and the crude product was extracted with dichloromethane (8 x 30 ml). The organic layers were dried over sodium sulfate and concentrated under reduced pressure. The crude product was purified over a glass filter with silicagel using 40% EtOAc in heptane as the eluens. This yielded 1.96 g (84%) of the pure product (S)-methyl-2-((S)-2-(*tert*-butoxycarbonylamino)-4-methylpentanamido)-4-methylpentanoate.
Step (2): The Boc protected dipeptide of step (1) was dissolved in DCM / TFA (44:11 ml) and stirred for 1.5 h. After working up a small sample with sat NaHCO₃ TLC showed that the reaction was complete. The reaction mixture was concentrated under reduced pressure yielding 2.58 g of a yellowish oil. ¹H NMR showed that the product must also contain some free trifluoroacetic acid along with the product, attempts to remove this by distillation failed and therefore the crude product (S)-methyl-2-((S)-2-amino-4-methylpentanamido)-4-methylpentanoate 2,2,2-trifluoracetate was used without further purification.
Step (3): The amino trifluoroacetate salt of step (2) was dissolved in dichloromethane (150 ml). At 0°C DIPEA was added, followed by HATU and Boc-Leu-OH. The mixture was stirred at room temperature for 2 h. According to TLC, the reaction was complete. The mixture was quenched with saturated ammonium chloride, and the water layer was extracted with dichloromethane (8 x 50 ml). The combined organic layers were dried over sodium sulfate and concentrated under reduced pressure. The crude product was purified over silicagel using EtOAc in heptane (4:6) as the eluens. The resulting tripeptide (6S, 9S, 12S)-methyl-6,9-diisobutyl-2,2-14-trimethyl-4,7,10-trioxo-3-oxa-5,8,11-triazapentadecane-12-carboxylate was obtained in a yield of 1.78 g (3.77 mmol; 50%).
Step (4): The Boc protected tripeptide of step (3) was dissolved in DCM /TFA (44:11 ml) and stirred for 1.5 h. After working up a small sample with saturated NaHCO₃ TLC showed that the reaction was complete (the product does not run with 50% EtOAc in heptane). The reaction mixture was concentrated under reduced pressure and stripped once with dichloromethane. Diethylether was added (20 ml) to the product for trituration, after 5 minutes, 10 ml heptane were added and the solid was filtered off after 40 minutes yielding 1,480 g of a white powder ((S)-methyl-2-((S)-2-amino-4-methylpentanamido)-4-methylpentanamido)-4-methylpentanoate 2,2,2-trifluoracetate).
Step (5): To a mixture of the tripeptide-TFA salt of step (4), N-methyl-piperazinylacetic acid, EDC and HOAt in toluene (50 ml, thought it was dichloromethane) DIPEA was added, and the mixture was stirred for 1 h during which a yellow solid was formed on the bottom of the flask. The mixture was quenched with saturated sodium bicarbonate (70 ml). The two phases were separated, and the water phase was extracted with dichloromethane (4 x 50 ml). The combined organic layers were dried over sodium sulfate and concentrated under reduced pressure yielding 0.203 g of crude material, which contained both the desired product as well as the starting material (according to LC-MS and ¹H NMR). The crude material was once more treated with EDC, HOAt and DIPEA in dichloromethane (25 ml) during 2 h. After work up (similar to the above described procedure) 0.231 g of crude material was obtained. According to LC-MS the reaction was complete. Purification over silica gel with gradient from 5-10% 0.5 M NH₃ in MeOH in dichloromethane gave 0.158 g of product as a crystalline white solid ((S)-methyl-4-methyl-2-((S)-4-methyl-2-((S)-4-methyl-2-(2-(4-methylpiperazin-1-yl)acetamido)pentanamido)-pentanamido)pentanoate).
Step (6): The starting material of step (5) was dissolved in Tesser's base (dioxane/MeOH/aqueous 4 N NaOH 15:4:1), and the mixture was stirred at room temperature. After one night of stirring the solution was concentrated yielding the crude product sodium (S)-4-methyl-2-((S)-4-methyl-2-((S)-4-methyl-2-(2-(4-methylpiperazin-1-yl)acetamido)pentanamido)pentanamido)pentanoate. This compound was used in the next step without further purification Na-analysis was performed..
Step (7): To a suspension of the crude product of step (6) in dichloromethane (5 ml) at 0°C NHS and EDC were sequentially added, and the mixture was stirred at 0°C for 3 hrs. The mixture was concentrated yielding a sticky solid. The sticky solid was re-dissolved in dichloromethane (7 ml). Then, diethylether (7 ml) was added, and the solvent was evaporated until half the volume. This addition of diethylether followed by partial evaporation was repeated twice after which the precipitate (ethyl (N,N-dimethylamino)propyl-ureum and sodium chloride) was filtered. The filtrate was concentrated yielding 52 mg of a viscous oil. When looking at the ¹H NMR spectrum it seems that this fraction may contain the desired succinate ester ((S)-2,5-dioxopyrrolidin-1-yl-4-methyl-2-((S)-4-methyl-2-((S)-4-methyl-2-(2-(4-methylpiperazin-1-yl)acetamido)-pentanamido)pentanamido)pentanoate) that is contaminated among others with ethyl diisopropyl ureum. The precipitate that was filtered (33 mg) probably contains the desired product. (singlet at 2.65 ppm, succinate protons).
Step (8): The starting material of step (6) was dissolved in Tesser's base (dioxane/MeOH/aqueous 4 N NaOH 15:4:1), and the mixture was stirred at room temperature. The reaction was monitored by LC-MS (acidic mode). After 2 h of stirring the solution was concentrated yielding the crude product. This compound was used in the next step without further purification. The yield was not determined
Step (9): To a solution of crude product of step (8) in a 1:1 mixture of dioxane (dry) and dichloromethane (2.0 ml, dried) at room temperature NHS, DIPEA (26 µl) and EDC (18.9 mg) were sequentially added, and the mixture was stirred at room temperature for 5 hours. 6-amino caproic acid and 2 ml dioxane (dry) were added. The mixture was stirred overnight. On the next day, more EDC (19 mg) was added to generate the activated succinimide ester. The mixture was again stirred over night. On the next day, a sample was taken from the reaction mixture, n-butylamine was added to the sample, and the resulting mixture was stirred for 10 minutes. The solution was concentrated under reduced pressure and analyzed by LC-MS: The crude product consisted of expected butylamide from the caproic acid adduct ([M+H]+ at m/z = 666.4), the caproic acid addition product ([M+H]+ at m/z=611.4), the succinimide ester from the starting material ([M+H]+ at m/z=553.4) and its corresponding methylester ([M+H]+ at m/z=512.4). The reaction mixture was quenched in saturated sodium bicarbonate (25 ml), and water (5 ml) was added. The aqueous phase was extracted with dichloromethane (5 x 20 ml), and the combined organic layers were dried over sodium sulfate and concentrated under reduced pressure. This resulted in 41 mg of solid. The solid was analyzed with LC-MS5 to reveal that it was a mixture of compounds. A sample was taken to perform a test reaction with butyl amine (see above). The product from this test reaction was analyzed with LC-MS5. It contains the expected butyl amide but its abundance seems to be lower than in the first test reaction. Due to the complexity of the mixture and to the uncertainty about composition of the product along with its potential instability. it was decided not to ship this product.
Step (10): The product of step (9) was purified by means of preparative HPLC. This yielded 8 mg of the free acid
Step (11): To a solution of starting material of step (10) in DMF EDC and N-hydroxysuccinimide (NHS) were added, and the mixture was stirred over night under N₂ atmosphere. On the next day, more NHS (4 mg) was added, and the mixture was again stirred for 5 h. A test sample was taken and allowed to react with excess N-butylamine for analysis. LC-MS of this sample showed that the product was formed (butyl amide with m/z = 667). The mixture was concentrated under reduced pressure and re-dissolved in dichloromethane (20 ml). This solution was quenched with saturated NaHCO₃ (20 ml).
   The organic layer was washed with saturated NaCl and dried over sodium sulfate. A test sample was taken for analysis: Excess butylamine was added, and the mixture was stirred for 15 minutes. The solution was concentrated under reduced pressure and analyzed by LC-MS. This analysis showed that the product was of good quality. The solution was concentrated to yield 7 mg of the desired (Leu)₃ product.

### Example 2 - Labeling of peptides using a (Leu)₃ labeling reagent

In a pilot experiment the (Leu)₃ compound shown in Fig. 6A was used as a lebeling reagent for labeling bovine serum albumin (BSA). Fig. 6A schematically illustrates the (Leu)₃ compound comprising both the reporter ion and the balance group of the isobaric label component as well as a NHS-ester as the reactive group. Fragmentation sites in tandem mass spectrometry (MS/MS) are indicated by arrows.

For labeling, 50 mg bovine serum albumin was reduced (5 mM TCEP, incubation for 15 min at 56°C) and alkylated (10 mM iodoacetamide, incubation for 30 min at room temperature in the dark); A batch of MC label (400 mg) was dissolved in 80 ml ethanol (3 ml were taken for separate mass spectrometric analysis of the label, cf. Fig. 6B) and subsequently added to the reduced and alkylated BSA. This mixture was incubated at room temperature for 2 hours.

After labeling, the buffer of the reaction mixture was exchanged to 100 mM ammonium bicarbonate, pH 8 using a 5K MWCO (molecular weight cut-off) ultrafiltration filter. Trypsin was added in a 1:25 ratio, and this mixture was incubated over night at 37°C.

For peptide identification, the digested BSA peptides were fractionated (sorted) using reversed phase liquid chromatography-electrospray ion trap mass spectrometry (LC-MS; cf. Fig. 6C, top panel). A representative LC-MS peak was further analyzed using MS (Fig. 6C, middle panel).A representative MS peak corresponding to a labeled peptide was subsequently subjected to MS/MS analysis (Fig. 6C, bottom panel). The resulting MS/MS spectra were then converted to an mgf-file (Mascot Generic Format) which was searched against the SwissProt database using the Mascot software. Next to methionine oxidation and cysteine carbamidomethylation, lysine MC-L3 was taken into account as a possible modification. Finally, the amino acid sequence (single-letter code) of the peptide analyzed was determined to be CCTKPESER (Fig. 6D). In another experiment (data not shown), a second BSA peptide having the amino acid sequence CASIQKFGER could be successfully identified using the (Leu)₃ labeling reagent.

### Example 3 - Biomarker identification in prostate cancer

Prostate cancer (PCa) is the most common malignancy in European males. In 2002, in about 225000 men were PCa was newly diagnosed and about 83000 men died from this disease. PCa is diagnosed by histological examination of prostate tissue that is obtained by ultrasound guided transrectal biopsy. Indications for biopsy are predominantly an increased serum prostate specific antigen (PSA) and/or an aberrant digital rectal examination. PSA is the standard diagnostic and prognostic PCa marker. PCa awareness, leading to widespread use of PSA testing has led to a lower tumor stage and grade at the time of diagnosis.

However, the use of PSA is associated with certain drawbacks. First, an increase in PSA can reflect a benign as well as a malignant prostate disease, i.e. PSA is not cancer specific, resulting in a negative biopsy rate of 70-80%. Thus, a large number of patients is unnecessarily undergoing prostate biopsies. Such increased detection leads to the diagnosis of clinically irrelevant tumors and potentially over-treatment. Furthermore, there is also the problem of patients having a clinically localized disease, who have undetected micro-metastases leading to symptomatic cancers in due time. Currently, these patients are not curable. Finally, many PCa patients who are not cured by radical therapy will ultimately develop hormone independence and treatment resistance. Early recognition of androgen independence may be an indication for systemic treatment by means of secondary treatments at an early stage of tumor progression. However, at present it is not possible to predict resistance against therapy at an early stage.

Accordingly, there is a need for PCa markers whose application result in an increase of diagnostic specificity, enable the differentiation between harmless and aggressive tumors, and allow the identification of progression towards androgen independence at an early disease stage.

Serum and urine contain degradation products of benign and malignant cells and their secreted products. The determination of biomarkers in these body fluids has certain advantages over the use of tissue markers. Even though the release of PCa-related proteins and/or peptides into serum and urine incompletely reflects tumor metabolism, these body fluids can easily be obtained. In contrast, prostate tissue sampling requires an invasive procedure (i.e. transrectal ultrasound-guided biopsy).

### Example 4 - Identification of potentially treatable tumors

Serum prostate-specific antigen (PSA) testing followed by transrectal ultrasound-guided needle biopsy allows the detection of prostate cancer in some men who might benefit from radical intervention (between 50 and 70 years). Prostate cancer can be detected in 2% (Frankel, S. et al. (2003) Lancet 361, 1122-28) to 40% of the cases (Thompson, I.M. et.al. (2003) New Engl. J. Med. 349, 215-24), depending on the intensity of the screening effort (e.g., number of PSA tests performed, level of PSA cutoff value, number and frequency of biopsies obtained). Since PSA is not prostate cancer-specific, a large number of false-positives results occur (cancer is not found in around 70% of men with raised PSA levels who undergo biopsy). The simple PSA blood test is safe and acceptable, but biopsy can be uncomfortable or painful and carries the risks of bleeding and infection. In addition, there will be an unpredictable number of false-negatives who later develop prostate cancer in the presence of a 'normal' PSA test: 36.5% of detectable tumors were identified in men who had PSA levels below 4ng/ml in the European Randomised Screening Trial (ERSPC) (Schröder, F.H. et al. (2000) J. Urology 163, 806-812).

It is clear that improved diagnostic tests for prostate cancer are required. PSA has been a useful tool to "trigger" biopsy and to monitor men with known cancer, but is not effective for screening. In the Prostate Cancer Prevention Trial where men had biopsies taken several times, regardless of PSA level, the incidence of prostate cancer was as shown below (Thompson, I.M. et al. (2004), *supra*): 6.6% with a PSA level of 0.5, 10.1% with a PSA level of 0.6 to 1.0, 17.0% with PSA level of 1.1 to 2.0, 23.9% with PSA level of 2.1 to 3.0, and 26.9% with PSA level of 3.1 to 4.0.

Even amongst those patients diagnosed with localized prostate cancer, there are further uncertainties. Likelihood and speed of tumor progression are currently impossible to predict. Tumors with high Gleason grades are more likely to progress than those with low grades. However, the risk to die within 15 years of diagnosis ranges from 60-80% for those patients suffering from tumors having the highest scores of 8-10 to 4-7% for those patients suffering from tumors having scores of 2-4, and 18-30% for those patients suffering from tumors having the most commonly screen-detected score of 6 (Albertsen, P.C. et al. (1998) JAMA 280, 975-80).

Current screening techniques therefore enable tumors to be but as it is currently impossible to distinguish between indolent and life-threatening lesions there is the potential for considerable over-treatment of insignificant disease.

### Example 5 - Parallel screening of patient samples for biomarker discovery

In order to determine clinically relevant protein expression differences between different samples, an experimental design as illustrated in Fig. 2 is used according to the present invention.

The experimental design is chosen such that any expression difference, which appears on the level of mass spectroscopy (MS) between the respective "light" and "heavy" isotopic label components reflect a potentially relevant discriminating feature between samples from a healthy/control donor or a benign disease *versus* an affect cancer patient in a local or advanced stage of the disease. The further analysis of the MS signals obtained by means of tandem MS/MS employing isobaric label components results in a more detailed answer concerning of differences in protein expression between the investigated groups.

In case a different clinical question is addressed, the design of the experiment has to be adjusted accordingly. For instance, if the goal is to identify markers for the staging of prostate cancer, the design of the experimental strategy may be the following:
- sample group 1: benign donor or intraepithelial neoplasia (PIN)
- sample group 2: local PCa disease
- sample group 3: locally advanced PCa disease
- sample group 4: aggressive, hormone-refractory PCa disease

After individually labeling the proteinaceous molecules of the samples to be analyzed using the labeling reagents of the invention, the samples are combined and the labeled proteinaceous molecules are separated by means of affinity purification via the isotopic label reagent/affinity tag comprised in the label.

The separated labeled proteinaceous molecules are further analyzed by mass spectrometric techniques.

## Claims

1. Labeling reagent for the labeling of proteinaceous molecules, comprising:
(a) an isobaric label component;
(b) an isotopic label component; and
(c) a reactive group capable of reacting with a proteinaceous molecule; wherein the isotopic label component concomitantly is an affinity tag.

2. The labeling reagent of claim 1, having a configuration, in which the isotopic label component/affinity tag is arranged between the isobaric label component and the reactive group.

3. The labeling reagent of claim 1, having a configuration, in which the isobaric label component is arranged between the isotopic label component/affinity tag and the reactive group.

4. The labeling reagent of any of claims 1 to 3, wherein the affinity tag is selected from the group consisting of (His)₆ tag, (His)₄ tag, (His)₃ tag, (His)₂ tag, (Leu)₄ tag, (Leu)₃ tag, (Leu)₂ tag, c-Myc tag, HA tag, FLAG tag, VSV-G tag, HSV tag, V5 tag, biotin and derivatives thereof, carbohydrates, and glycans.

5. The labeling reagent of any of claims 1 to 4, wherein the reactive group is selected from the group consisting of amino-reactive groups, sulfhydryl-reactive groups, and carboxyl-reactive groups.

6. Kit-of-parts, comprising a combinatorial plurality of n·m labeling reagents as defined in any of claims 1 to 5, wherein the integer n is ≥ 2 and represents the number of differentially labeled isobaric label components, and wherein the integer m is ≥ 2 and represents the number of differentially labeled isotopic label components/affinity tags.

7. The kit of claim 6, wherein the integer n is ≥ 4 and the integer m is ≥ 2.

8. Method for the analysis of one or more proteinaceous molecules in one or more samples, comprising:
(a) providing a kit-of-parts as defined in any of claims 5 to 7, the kit-of parts comprising a combinatorial plurality of n·m labeling reagents;
(b) labeling at least one subset of the proteinaceous molecules comprised in the one or more samples by individually contacting each of the one or more samples with another one of the n·m labeling reagents;
(c) combining the one or more samples;
(d) separating the labeled at least one subset proteinaceous molecules by affinity purification via the affinity tag comprised in the label; and
(e) analyzing the separated at least one subset of proteinaceous molecules.

9. The method of claim 8, being performed with M·N samples, wherein the integer M represents the number of groups of samples and the integer N represents the number of individual members of each group of samples, and wherein N = n and M = m.

10. The method of claim 8 or 9, further comprising: cleaving the proteinaceous molecules into peptides prior to performing step (b).

11. The method of any of claims 8 to 10, further comprising: fractionating the separated at least one subset of proteinaceous molecules prior to performing step (e).

12. The method of any of claims 8 to 11, wherein the analysis of the separated at least one subset of proteinaceous molecules is performed by means of mass spectrometry.

13. The method of any of claims 8 to 12, further comprising: comparing the results of the analyses obtained in step (e) for each of the one or more samples.

14. The method of any of claims 8 to 13, wherein the method is performed in a high-throughput format.

15. Use of a labeling reagent of any of claims 1 to 5 and/or a kit-of-parts of any of claims 6 or 7 for performing protein expression profiling or for performing proteomic analyses.
